# EUROPEAN PATENT SPECIFICATION

(11) **EP 2 224 947 B1**
(45) Date of publication and mention of the grant of the patent: **18.03.2015**
(21) Application number: 08854557.9
(22) Date of filing: 25.11.2008
(51) Int. Cl.: A61K 38/31, A61P 35/00

(54) **USE OF SOMATOSTATIN ANALOGS IN MENINGIOMA**
VERWENDUNG VON SOMATOSTATIN ANALOGA ZUR BEHANDLUNG VON MENINGIOMEN
UTILISATION D'ANALOGUES DE SOMATOSTATINE DANS LE MÉNINGIOME

(30) Priority: 28.11.2007 US 4549 P
(43) Date of publication of application: 08.09.2010
(73) Proprietor: Novartis AG, 4056 Basel (CH)
(72) Inventor: GRUIA, Gabriela, Hoboken New Jersey 07030 (US); TAVORATH, Ranjana, Short Hills New Jersey 07078 (US)
(74) Representative: Gruber, Markus
(86) International application number: PCT/US2008/084598
(87) International publication number: WO 2009/070551

(56) References cited:
- WO-A-02/10192
- RÜNZI M W ET AL: "Successful treatment of meningioma with octreotide." LANCET 13 MAY 1989, vol. 1, no. 8646, 13 May 1989 (1989-05-13), page 1074, XP002518036 ISSN: 0140-6736
- SHIMATSU A ET AL: "Meningioma in an acromegalic patient treated with long-term octreotide therapy" FRONTIERS IN NEUROENDOCRINOLOGY, ACADEMIC PRESS, SAN DIEGO, US, vol. 27, no. 1, 1 May 2006 (2006-05-01), page 89, XP024922242 ISSN: 0091-3022 [retrieved on 2006-05-01]
- JAFFRAIN-REA M-L ET AL: "VISUAL IMPROVEMENT DURING OCTREOTIDE THERAPY IN A CASE OF EPISELLAR MENINGIOMA" CLINICAL NEUROLOGY AND NEUROSURGERY, ELSEVIER, AMSTERDAM, NL, vol. 100, no. 1, 1 March 1998 (1998-03-01), pages 40-43, XP000907228 ISSN: 0303-8467

## Description

The present invention relates to a new use of Somatostatin (SRIF) peptidomimetics (also referred to as Somatostatin- or SRIF-analogs) in the treatment of meningioma.

Somatostatin is a tetradecapeptide having the structure:

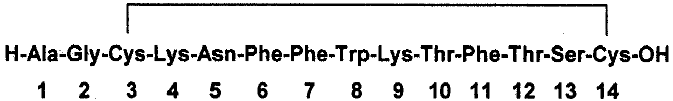

The somatostatin class is a known class of small peptides comprising the naturally occurring somatostatin-14 and analogues having somatostatin related activity, e.g. as disclosed by A.S. Dutta in Small Peptides, Vol.19, Elsevier (1993). By "somatostatin analog" as used herein is meant any straight-chain or cyclic polypeptide having a structure based on that of the naturally occurring somatostatin-14 wherein one or more amino acid units have been omitted and/or replaced by one or more other amino radical(s) and/or wherein one or more functional groups have been replaced by one or more other functional groups and/or one or more groups have been replaced by one or several other isosteric groups. In general, the term covers all modified derivatives of the native somatostatin-14 which exhibit a somatostatin related activity, e.g. they bind to at least one of the five somatostatin receptor (SSTR3), preferably in the nMolar range.

Natural somatostatin binds and activates all 5 somatostatin receptors (SSTR 1-5) with nmol efficacy and thus causes its multiple physiological effects.

Synthetically available somatostatin analogs differ in their binding affinity to the different somatostatin receptor subtypes and often bind selectively to one or few subtypes with significantly higher affinity.

A somatostatin analog of particular interest according to the present invention has a high binding affinity to human SSTR1,2,3,5 and have been described e.g. in WO 97/01579. The somatostatin (SRIF) peptidomimetic (also referred to as Somatostatin- or SRIF-analog) is pasireotide.

Pasireotide, also called cyclo[{4-(NH₂-C₂H₄-NH-CO-O-)Pro}-Phg-DTrp-Lys-Tyr(4-Bzl)-Phe], Phg meaning -HN-CH(C₆H₅)-CO- and Bzl meaning benzyl, is for instance disclosed in WO02/10192 and is represented by the following formula:

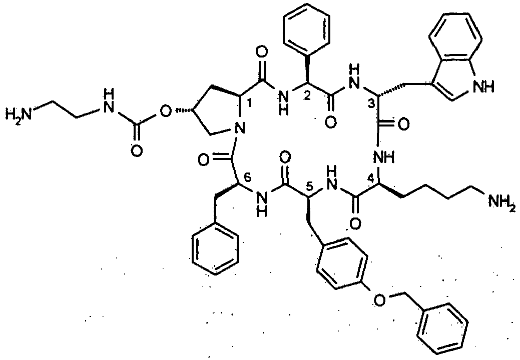

In WO02/10192 is also disclosed a synthesis for and uses of pasireotide.

Pasireotide has been shown to have a inhibitory effect on the secretion of several hormones (e.g. GH, GH dependent and GH independent IGF-1 secretion, ACTH, cortisol resp. corti-costerone) and can be used, for instance, in the treatment of disorders with an aetiology comprising or associated with excess GH-secretion and/or excess of IGF-1.

Meningiomas are very common and (in about 90% of the cases) histologically benign intradural, extra-axial primary brain tumors which develop from neoplastic meningothelial (arachnoidal cap) cells, and which, after gliomas, are the most common primary brain tumor in adults.

Aggressive surgical resection is the treatment of choice for meningiomas, and when clinically and anatomically feasible, complete removal of tumor offers the best chance of cure. Unfortunately, the condition of the patient or the location of the tumor is often not compatible with gross total removal.

In the absence of complete surgical resection, however, tumor recurrence is, over time, common despite conventional or stereotactic radiation and five- and ten-year survival rates are disappointingly low.

Hormonal and chemotherapeutic treatment options rarely produce significant or durable tumor responses.

Meningiomas are tumors with a high frequency of surface somatostatin receptors. It has been reported that the addition of somatostatin inhibits meningioma growth in vitro.

Treatment of meningioma with octreotide, an octapeptide somatostatin analog with a longer half-life than naturally occurring somatostatin of approximately 1.5 hours, which can be given subcutaneously, in a very limited number of patients with unclear results has been described (Jaffrain-Rea ML, Minniti G, Santoro A, Bastianello S, Tamburrano G, et al., Clin Neurol Neurosurg 100:40-43, 1998; Garcia-Luna PP; Relimpo F, Pumar A, et.al., .J Neurosurg Sci 37: 237-241, 1993; Runzi MW, Jaspers C, Windeck R, Benker G, Mehdorn M, et al., Lancet 2:217-8, 1989, 17).

Furthermore, in a recent pilot study in 16 patients with multiply recurrent, treatment-refractory meningiomas octreotide has been studied with encouraging response and toxicity data (Glantz MJ, Fadul CE, Chambarlain MC., Neurology 69: 969-973).

However, octreotide is a relatively large lipophilic molecule (consisting of 8 amino acids) which preferentially binds to SSTR2 and, only to a lesser extent, to SSTR3 and SSTR5 having a half-life of only 1.5 hours.

Given the limitations of the available agents, some patients do not have an acceptable abortive treatment option.

There is therefore a compelling need to develop new pharmacological approaches to effectively and safely treat meningioma patients.

Surprisingly, it has been found that the compound according to the present invention, which has a high binding affinity to several SSTR, especially SSTR1,2,3,5, pasireotide, has a beneficial effect in the treatment of meningioma, including recurrent or progressive meningiomas.

It can be surprisingly shown that both the efficacy and tolerability of pasireotide (having an extended half life of 11 hours) in patients with recurrent meningiomas is better than that observed with other treatments, including octreotide, for instance.

The term "SRIF-analog with a high binding affinity to human SSTR1,2,3,5" as used herein (also referred to as COMPOUND OF THE INVENTION) refers to compounds which have a high binding affinity to SSTR1, SSTR2, SSTR3 and SSTR5, preferentially an IC50 < 10 nmol/l at SSTR1 and SSTR2 and an IC50 < 3 nmol/l at SSTR3 and SSTR5; (Schmid et al., Neuroendocrinol. 2004;80:47-50). The COMPOUND OF THE INVENTION is pasireotide or a pharmaceutically acceptable salt thereof.

The term "treatment" as used herein comprises the treatment of patients having meningioma which effects the delay of progression of the disease in said patients or leads to patients with stable, i.e. clinically unchanged, disease or to responding patients, i.e. with a decrease in tumor size.

It can be shown by established test models that the use of COMPOUND OF THE INVENTION, pasireotide, results in an effective treatment of meningioma.

In accordance with the particular findings of the invention, the present invention also provides a composition for use in method of treating meningioma in a subject in need thereof comprising administering to said subject a therapeutically effective amount of the COMPOUND OF THE INVENTION, pasireotide, or a pharmaceutically acceptable salt thereof.

In a further aspect, the present invention relates to the use of the COMPOUND OF THE INVENTION, pasireotide, or a pharmaceutically acceptable salt thereof for the preparation of a pharmaceutical composition for the treatment of meningioma.

The present invention relates also to a pharmaceutical composition for use in treatment of meningioma, comprising a therapeutically effective amount of the COMPOUND OF THE INVENTION, pasireotide, or a pharmaceutically acceptable salt thereof, together with one or more pharmaceutically acceptable diluents or carriers.

Also disclosed herein is a commercial package comprising a COMPOUND OF THE INVENTION, pasireotide, together with instructions for use thereof in the treatment of meningioma.

Pharmaceutical compositions for the treatment of meningioma comprise an effective amount of a COMPOUND OF THE INVENTION, pasireotide, in free base form or in pharmaceutically acceptable salt form together with one or more pharmaceutically acceptable diluent or carrier. Such compositions may be formulated in conventional manner. A COMPOUND OF THE INVENTION, pasireotide, may also be administered in sustained release form, e.g. in the form of implants, microcapsules, microspheres or nano-spheres comprising e.g. a biodegradable polymer or copolymer, in the form of a liposomal formulation, or in the form of an autogel, e.g. a solid or semi-solid composition capable of forming a gel after interaction with patient's body fluids.

The COMPOUND OF THE INVENTION, pasireotide, can, for example, be formulated as disclosed in WO05/046645.

COMPOUND OF THE INVENTION, pasireotide, or a pharmaceutically acceptable salt thereof may be administered by an conventional route, for example parenterally e.g. in form of injectable solutions or suspensions (including e.g. the sustained release form as indicated above), orally using a conventional absorption enhancer if necessary, in a nasal or a suppository form or topically, e.g. in the form of an ophthalmic liquid, gel, ointment or suspension preparation, e.g. a liposomal, microsphere or nanosphere formulation, e.g. for instillation or subconjunctival or intra- or peri-ocular injections.

The present pharmaceutical compositions are prepared in a manner known per se, and comprise approximately from 1 % to 100 %, preferentially from approximately 1 % to 40 %, especially from approximately 20 % to 30%, active ingredient.

The structure of the active ingredients identified by code nos., generic or trade names may be taken from the actual edition of the standard compendium "The Merck Index" or from databases, e.g. Patents International (e.g. IMS World Publications). Any person skilled in the art is fully enabled to identify the active ingredients and, based on these references, likewise enabled to manufacture and test the pharmaceutical indications and properties in standard test models, both *in vitro* and *in vivo*.

It will be understood that in the discussion of compositions for use, references to the active ingredients are meant to also include the pharmaceutically acceptable salts. If these active ingredients have, for example, at least one basic center, they can form acid addition salts. Corresponding acid addition salts can also be formed having, if desired, an additionally present basic center. The active ingredients having an acid group (for example COOH) can also form salts with bases. Salts include acid addition salts with e.g. inorganic acids, polymeric acids or organic acids, for example with hydrochloric acid, acetic acid, lactic acid, aspartic acid, benzoic acid, succinic acid or pamoic acid. Acid addition salts may exist as mono- or divalent salts, e.g. depending whether 1 or 2 acid equivalents are added to the COMPOUND OF THE INVENTION in free base form. Preferred salts according to the present invention are salts of pasireotide.

Preferred salts for pasireotide are the lactate, aspartate, benzoate, succinate and pamoate including mono- and di-salts, more preferably the aspartate di-salt and the pamoate mono-salt.

The active ingredient or a pharmaceutically acceptable salt thereof may also be used in form of a hydrate or include other solvents used for crystallization.

The person skilled in the pertinent art is fully enabled to select a relevant test model to prove the hereinbefore and hereinafter indicated therapeutic indications and beneficial effects.

The pharmacological activity of the COMPOUND OF THE INVENTION, pasireotide, in meningioma may, for example, also be demonstrated in clinical studies.

The effective dosage of the active ingredients employed may vary depending on the particular compound or pharmaceutical composition employed, the mode of administration, the severity of the condition being treated. Thus, the dosage regimen is selected in accordance with a variety of factors including the route of administration and the renal and hepatic function of the patient. A physician, clinician or veterinarian of ordinary skill can readily determine and prescribe the effective amount of the single active ingredients required to prevent, ameliorate or arrest the progress of the condition. Optimal precision in achieving concentration of the active ingredients within the range that yields efficacy without toxicity requires a regimen based on the kinetics of the active ingredients' availability to target sites. This involves a consideration of the distribution, equilibrium, and elimination of the active ingredients.

The efficacy of pasireotide in the treatment of meningioma is shown in a single-arm, phase II trial in patients with documented recurrent or progressive intracranial meningioma who have failed conventional therapy and are not candidates for complete surgical resection of their tumors and/or radiation at the time of study entry. Patients receive pasireotide subcutaneously at a dose of 1200 µg twice daily. One treatment cycle is defined as four weeks of therapy. Complete blood counts are obtained, and neurologic examinations and contrast-enhanced cranial MR scans are performed.

Patients will continue treatment until progressive disease is documented, development of an unacceptable toxicity, patient/investigator request to discontinue treatment, or death.

## Claims

1. Pasireotide or a pharmaceutically acceptable salt thereof for use in the treatment of meningioma.

2. A pharmaceutical composition for use in the treatment of meningioma, comprising pasireotide or a pharmaceutically acceptable salt thereof, together with one or more pharmaceutically acceptable diluents or carriers therefore.

## Patentansprüche

1. Pasireotid oder ein pharmazeutisch verträgliches Salz davon zur Verwendung bei der Behandlung von Meningeom.

2. Pharmazeutische Zusammensetzung zur Verwendung bei der Behandlung von Meningeom, umfassend Pasireotid oder ein pharmazeutisch verträgliches Salz davon, zusammen mit einem oder mehreren pharmazeutisch verträglichen Verdünnungsmitteln oder Trägerstoffen dafür.

## Revendications

1. La pasireotide ou un sel pharmaceutiquement acceptable de celle-ci pour son utilisation dans le traitement du méningiome.

2. Une composition pharmaceutique pour son utilisation dans le traitement du méningiome, comprenant la pasireotide ou un sel pharmaceutiquement acceptable de celle-ci, avec un ou plusieurs diluants ou véhicules pharmaceutiquement acceptables de celle-ci.
